# EUROPEAN PATENT APPLICATION

(11) **EP 0 818 431 A1**
(43) Date of publication of application: **14.01.1998**
(21) Application number: 97304047.0
(22) Date of filing: 11.06.1997
(51) Int. Cl.: C07B 61/00, C07C 209/24, C07C 209/28, C07C 209/52, C07C 209/84, G01N 33/50, B01L 3/00, B01J 19/00, C07D 209/16, C07C 323/32, C07D 211/56, C07D 307/14, C07C 211/27, C07C 215/50, C07C 211/30, C07D 401/12, C07D 213/38, C07D 405/12

(54) **Scavenger assisted combinatorial process for preparing libaries of secondary amine compounds**

(30) Priority: 14.06.1996 US 17848 P
(71) Applicant: ELI LILLY AND COMPANY, Indianapolis, Indiana 46285 (US)
(72) Inventor: Kaldor, Stephen Warren, Indianapolis, Indiana 46254 (US); Siegel, Miles Goodman, Indianapolis, Indiana 46260 (US); Fritz, James Erwin, McCordsville, Indiana 46055 (US)
(74) Representative: Hudson, Christopher Mark

(57) **Abstract**

This invention relates to a novel solution phase process for the preparation of secondary amine combinatorial libraries. These libraries have use for drug discovery and are used to form wellplate components of novel assay kits.

## Description

This invention relates to a solution phase synthesis of combinatorial libraries of secondary amines. These libraries are useful for discovery of lead compounds for drug development.

A known method of preparing amines is by reductive amination. Thus, aldehydes or ketones are used to prepare primary, secondary and tertiary amines as follows:

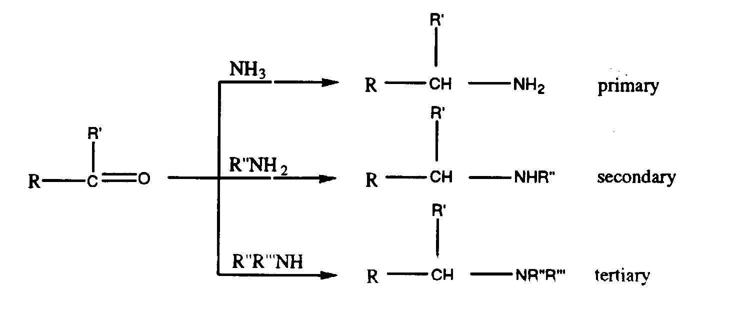

Research and development expenses account for the largest outlay of capital in the drug industry. Synthesis of compounds is an expensive and time consuming phase of research and development. Historically, research chemists individually synthesized and analyzed hundreds of high purity compounds for biological screening to develop pharmaceutical leads. Although past methods brought new drugs to market, the limitations of individual synthesis and insistence on compound characterization considerably slowed the discovery process.

The need for more rapid and less expensive drug discovery methodology is increasingly important in today's competitive drug industry.

More recently, modern drug discovery has used the methods of combinatorial chemistry to generate large numbers (viz., about 10² to 10⁶) of compounds generically referred to as "libraries." An important objective of combinatorial chemistry is to generate lead compounds for pharmaceutical research.

Theoretically, the total number of compounds which may be produced for a given library is limited only by the number of reagents available to form substituents on the variable positions on the library's molecular scaffold. The combinatorial process lends itself to automation, both in the generation of compounds and their biological screening.

Combinatorial chemistry may be performed in a manner where libraries of compounds are generated as mixtures with complete identification of individual compounds postponed until after positive screening results are obtained. However, a preferred form of combinatorial chemistry is "parallel array synthesis" where individual reaction products (most often individual compounds) are synthesized together, but are retained in separate vessels. For example, the library compounds are held in the individual wells of 96 well microtiter plates. Use of standardized microtiter plates or equivalent apparatus is advantageous because such an apparatus is readily manipulated by programmed robotic machinery.

Conventionally, combinatorial chemistry is conducted on a solid phase support, normally a polymer. The library scaffold is cleavably tethered to the solid support by a chemical linker. Reactions are carried out to modify the scaffold while tethered to the solid support. In a final step, the product is cleaved and released from the solid support. A general procedure for conventional solid phase synthesis is illustrated by the following scheme where the shaded circle symbol is, for example, a high molecular weight polymer:

### Solid-Phase Synthesis:

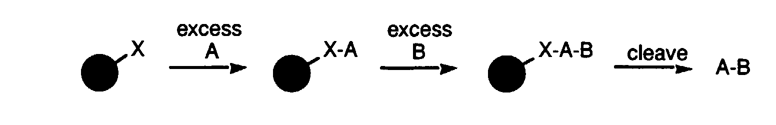

Variations in reagents (e.g., "A", "B", in the general scheme, supra.) produce the desired structural diversity. Separation of solid phase product and unreacted soluble reactant is done by simple separation techniques such as filtration, decantation, or washing. These separation solid phase synthesis techniques have general applicability to a wide variety of combinatorial reactants and lend themselves to large scale simultaneous/automated library creation.

The rate determining step in small molecule synthesis is typically not actual construction of the desired new chemical entities. Rather, the difficulty of synthesis is frequently caused by the task of isolating reaction product from unreacted starting materials, by-products or other impurities.

Unfortunately, it is not always practicable to tether a desired combinatorial scaffold to a solid support. A significant number of combinatorial reaction schemes are desirably done in solution phase. Moreover, not all desired solution phase reactions are driven to completion using near stoichiometric ratios of reactants. Frequently, one reagent is added in considerable excess to drive a solution phase reaction to completion. The result is a reaction medium with soluble product and soluble unreacted co-reactant. Consequently, traditional organic synthetic methods often require complex purification strategies which limit their use, particularly in combinatorial syntheses.

Polymeric reagents have been known and used for general chemical synthesis in various roles; such as follows:
a) The article, *Cyanoborohydride supported anion exchange resin as a selective reducing agent* by Hutchins, Robert O.: Natale, Nicholas R. and Taffer, Ira M.; J.C.S. Chem Comm., pg. 1088-9, 1978 describes various reactions including reductive amination using cyanoborohydride anion on ion-exchange resin. The spent resin reagent is removed by simple filtration and washing.
b) The article, *Synthesis and Reactivity of Polymer-Supported Reducing Agents with Chemically Modified Surfaces* by Menger, Fredric M., Hiraku, Shinozaki, and Lee, Hsueh-Chi; J. Org. Chem 1980, 45, 2724-2725 describes borohydride and cyanoborohydride functional anion exchange resins for carbonyl group reduction. Aldehydes and ketones are reduced to form alcohols by use of polymeric reagents.
c) US Patent No. 3,873,621 describes reductive amination reactions carried out using alkali-metal and quaternary ammonium cyanoborohydrides.
d) The article, *The reduction of α,β-unsaturated nitroalkenes to nitroalkanes with borohydride supported on an ion exchange resin,* by Goudgaon, Naganna, M.; Wadgaonkar, Prakash P.; and Kabalka, George W.; Synthetic Communications, 19(5&6), 805-811 (1989) describes the use of polymer supported borohydride reagent used to reduce nitroalkenes to nitroalkanes.
e) The article, *Borohydride reducing agent derived from anion exchange resin: Selective reduction of α,β-carbonyl compound* by Sande, A.R. et. al., Tetrahedron Letters, Vol. 25, No. 32, pp 3501-3504 1984 describes the use of borohydride exchange resin for the reduction of cyclic and acyclic ketones to alcohols with the attendant advantage of simple separation by filtration to give product free of boron moiety.
f) The article, A *reductive amination/lactamization procedure using borohydride reagents* by Abdel-Magid, Ahmed F.; Harris, Brice D. and Maryanoff, Cynthia A.; Synlett, pgs. 81-3, January 1994 describes reductive amination of carbonyl compounds using sodium triacetoxyborohydride.
g) The article, *New Probes for the Study of Acylation Reactions,* by J. Rebek, D. Brown, and S. Zimmerman (Contribution No. 3481), Journal of the American Chemical Society, 97:15, p.4408, July 23, 1975, JACS 97:15 July 23, 1975 describes the use of polymer bound isocyanate to activate carboxylic acid.
h) The article, *Chemical Modification of Polymers. Borohydride Reducing Agents Derived from Anion Exchange Resins,* by H. W. Bibson and F.C. Bailey, J.C.S. Chem. Comm., p. 815, 1977 describes the preparation of an insoluble polymer bound reducing agent by reacting anion exchange resins of the quaternary ammonium type with aqueous NaBH₄.
i) The article, *Solid Phase Synthesis of Oligosaccharides. I. Preparation of the Solid Support. poly(p-(1-propen-3-ol-1-yl) styrene,* by J. M. Frechet and C. Schuerch, Journal of the American Chemical Society, 93:2, p. 492-496 describes the preparation of -CHO functional polymers by contacting chloromethylated resin with methyl sulfoxide and sodium bicarbonate.
j) U.S. Patent No. 3,576,870 describes the purification of dimethylacetamide by removing acetic anhydride with a basic ion exchange resin containing primary or secondary amino groups.
k) The article, *Use of Polymeric Nucleophiles for the Selective Binding and Removal of α-Methylene-γ-butyrolactone Allergens from Complex Mixtures,* by A. Cheminat and C. Benezra, Tetrahedron Letters, Vol. 21, p. 617-619 (1980) describes an amine functional polymer used as a nucleophile for removal of an α,β-unsaturated lactone electrophile.
l) The article, *Polymeric De-blocking Agents for the Fluoren-9-ylmethoxycarbonyl (FMOC) Amino-protecting Group,* by L.S. Carpino and J.R. Williams, J.C.S. Chem. Comm., p.450-451, (1978) describes the use of a resin bound piperazine to remove FMOC with subsequent scavenging of the dibenzofulvene contaminant.
m) The article, *Piperazino-Functionalized Silica Gel as a Deblocking-Scavenging Agent for the 9-Fluorenylmethyloxycarbonyl Amino-Protecting Group,* by L.A. Carpino, E.M.E. Mansour, and J. Knapczyk, J. Org. Chem., 48, p.666-669 (1983) describes a silica bound piperazine to remove FMOC with subsequent scavenging of the dibenzofulvene contaminant.
n) The article, Preparation of High Capacity Aminomethyl-Polystyrene Resin, by C. C. Zikos and N.G. Ferderigos, Tetrahedron Letters, Vol. 36, No. 21, p. 3741-3744, 1995, describes the preparation of an amine functional resin.
o) U.S. Patent No. 5,244,582 relates to reactive groups immobilized on inorganic substrates such as glass. Such immobilized groups can be used to remove nitrosating agents in liquids, etc.

There remains a need to develop solution phase combinatorial processes for making libraries.

### Summary of the Invention

Combinatorial chemistry may be used at two distinct phases of drug development. In the discovery phase highly diverse libraries are created to find lead compounds. In a second optimization phase, strong lead compounds are much more narrowly modified to find optimal molecular configurations. The method of this invention has applicability for making diverse libraries of secondary amine compounds useful for finding new lead compounds, and directed libraries of secondary amines useful for optimizing a particular desired biological activity.

FIG. 1 is a top view of a wellplate apparatus.

FIG. 2 is a side view of a wellplate apparatus.

### Detailed Description of the Invention

### I. Definitions:

The following terms have the meaning defined below when used in this specification of the invention:

"Assay kit" means an assemblage of two cooperative elements, namely, (i) a wellplate apparatus, and (ii) biological assay materials.

"Biological assay materials" are materials necessary to conduct a biological evaluation of the efficacy of any library compound in a screen relevant to a selected disease state.

"Directed Library" is a collection of compounds created by a combinatorial chemistry process for the purpose of optimization of the activity of a lead compound, wherein each library compound has a common scaffold, and the library, considered in its entirety, is a collection of closely related homologues or analogues to the lead compound (compare to "Diverse library").

"Diverse library" means a library where the substituents on the combinatorial library scaffold are highly variable in constituent atoms, molecular weight, and structure and the library, considered in its entirety, is not a collection of closely related homologues or analogues (compare to "Directed library").

"Lead compound" means a compound in a selected combinatorial library for which the Assay kit has revealed significant activity relevant to a selected disease state.

"Library" is a collection of compounds created by a combinatorial chemical process, said compounds having a common scaffold with one or more variable substituents.

"Library compound" means an individual reaction product (usually a single compound) in a library produced by the method of the invention.

"Parallel array synthesis" means a method of conducting combinatorial chemical synthesis of libraries wherein the individual combinatorial library reaction products are separately prepared and stored without prior or subsequent intentional mixing.

"Reaction zone" means the individual vessel location where the combinatorial chemical library compound preparation process of the invention is carried out and individual library compounds synthesized. Suitable reaction zones are the individual wells of a wellplate apparatus.

"Scaffold" means the invariant region (viz., core) of the compounds which are members of a library (viz., secondary amine).

"Simultaneous synthesis" means making of library of compounds within one production cycle of a combinatorial method (not making all library compounds at the same instant in time).

"Solid-supported scavenger" means a reaction medium insoluble solid substance containing chemical functionality reactive with the soluble impurity (viz., usually excess reactant) desired to be removed from the reaction medium in the presence of soluble product.

"Substituents" are chemical radicals which are bonded to the scaffold through the combinatorial synthesis process. The different functional groups account for the diversity of molecules throughout the library and are selected to impart diversity of biological activity to the scaffold in the case of diverse libraries, and optimization of a particular biological activity in the case of directed libraries.

"Reagent" means a reactant, any chemical compound used in the combinatorial synthesis to place substituents on the scaffold of a library.

"Wellplate apparatus" means a structure capable of holding a plurality of library compounds in dimensionally fixed and defined positions.

"Non-interfering substituent", refers to a halo or organic (non-hydrogen) radical suitable for substitution as R₁, R₂, R₃ or R₄ in the reactants used in the process of making a combinatorial secondary amine library. Non-interfering substituents are those that do not significantly impede the solution phase processes of the invention or interfere with the use of a solid phase scavenger in said processes. Suitable non-interfering radicals include, but are not limited to, C₁-C₁₀ alkyl, C₂-C₁₀ alkenyl, C₂-C₁₀ alkynyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, -(CH₂)ₘ-O-(C₁-C₁₀ alkyl), heterocyclic radical, substituted heterocyclic radical, aryl, and substituted aryl.

"Cₓ-C_{y} alkyl" means a straight or branched chain hydrocarbon of between x and y carbon atoms.

"Cₓ-C_{y} cycloalkyl" means a ring of between x and y carbon atoms having at least one fully saturated bond.

"Aryl" means one or more aromatic rings, each of 5 or 6 carbon atoms. Multiple aryl rings may be fused, as in naphthyl, or unfused, as in biphenyl.

"Substituted Aryl" having one or more non-interfering groups as substituents.

"Halo" means chloro, fluoro, iodo or bromo.

"Heterocycle" means one or more rings of 5, 6, or 7 atoms with or without unsaturation or aromatic character and at least one ring atom which is not carbon. Preferred heteroatoms include sulfur, oxygen, and nitrogen. Multiple rings may be fused, as in quinoline or benzofuran.

"Substituted heterocycle" means heterocycle with one or more side chains formed from non-interfering substituents.

### II. General description of the secondary amine combinatorial library:

The library of the invention is a combinatorial library comprising individual secondary amine library compounds represented by the general formula (I): 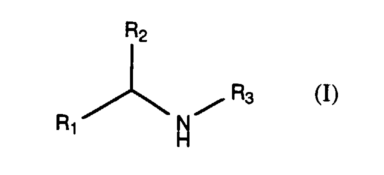 wherein R₁, R₂, and R₃ are as set out below.

### III. General description of the process for making the secondary amine combinatorial library of the invention:

Amines, including secondary amines, may be prepared by reductive amination with aldehydes or ketones, as follows:

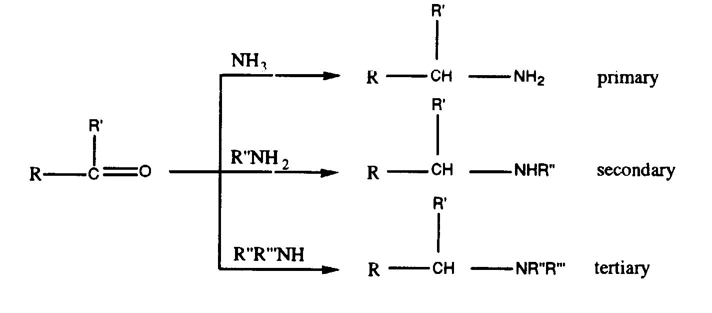

The reductive amination synthesis scheme for preparing individual secondary amines set out above is modified according to this invention to prepare secondary amine combinatorial libraries.

This invention is particularly well suited as a general method for preparing a structurally diverse secondary amine library. The final form of the library compounds in the secondary amine library may be as a solute dissolved in a solvent (viz., the reaction medium) or the solvent may be removed and the final product retained as a powder, paste or oil.

The secondary amine library compounds of this invention are non-peptide, substantially non-naturally occurring molecules having a molecular weight range of from about 100 to about 700.

The reaction zone for forming each secondary amine library compound of this invention contains a solvent. The solvent reaction medium is typically a solvent not only for the library compound desired, but also for impurities such as; (i) unreacted reagent, and/or (ii) by-product(s). The impurities in the reaction mixture are generally soluble in the solution phase since they frequently have a molecular weight lower than or equal to the product and share broad solubility characteristics of the product.

One method of driving a multiplicity of diverse reductive amination secondary amine forming reactions to completion (consumption of the aldehyde reactant) is to use a stoichiometric excess of amine reagent, preferably a large stoichiometric excess. For such methods the efficient removal of excess amine reagent may be accomplished with a solid supported aldehyde functional scavenger as taught herein.

Combinatorial techniques must be very robust to work well for highly diverse groups of reactants. The solid supported scavengers employed by the processes of this invention remove excess unreacted reactant in a manner readily adapted to the diversity of reagents employed in combinatorial chemistry, particularly parallel array synthesis.

A general scheme for the use of solid phase scavengers is as follows:

### Solid-Phase Scavengers:

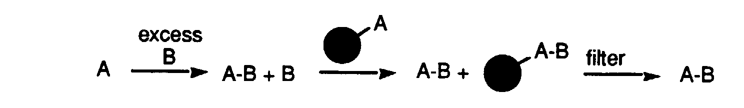

The solid supported scavenger is filtered from the liquid reaction media and the purified product A-B retained.

The utility of the method of the invention and the secondary amine library created thereby is for developing new drugs. Pharmaceutical drug discovery relies heavily on studies of structure-activity relationships wherein the structures of discovered "lead compounds" are the basis for new drug development. The method of the invention systematically and simutaneously generates large numbers of diverse secondary amine molecules useful as a source of lead compounds. The combinatorial secondary amine libraries of the invention may be screened for pharmacologically active compounds using conventional screen protocols known in the art for any targeted disease state.

The following section IV thru XI describe a combinatorial solution phase reductive amination process for making secondary amine libraries

### IV. Description of the scavenger assisted solution phase reductive amination process of making secondary amine combinatorial libraries of the invention.

This invention is a scavenger assisted combinatoric reductive amination process for preparing a library of compounds having a secondary amine scaffold with three variable substituents, said compounds represented by the formula; 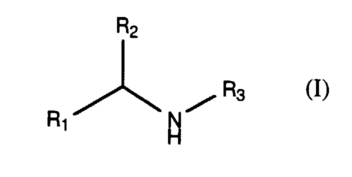 said process comprising the steps of:
a) adding to each reaction zone containing a liquid medium (n) equivalents of a carbonyl functional reactant represented by the formula: 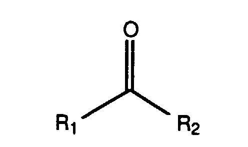 where R₁ is a non-interfering substituent and R₂ is H or a non-interfering substituent;
b) adding to each reaction zone of step (a) at least 1.1(n) equivalents of a solvent soluble primary amine reactant;

   R₃-NH₂

   where R3 is a non-interfering substituent and maintaining the reaction zone at a temperature and for a time sufficient to permit reaction of the first and second reactants to yield an imine (II); 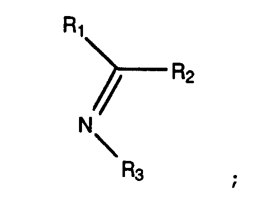
c) adding to each reaction zone a reducing agent and maintaining each reaction zone at a temperature and for a time sufficient to permit imine reduction;
d) adding to each reaction zone of step (c) a solid supported aldehyde functional scavenger represented by the formula; 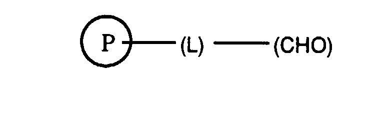 wherein; 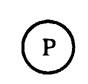 is a solid-support insoluble in the liquid reaction medium of the reaction zone, -(L)- is a divalent linking group, and (CHO) is an aldehyde functional substituent; and adding said scavenger in an amount wherein the equivalents of (CHO) are at least equal to the excess equivalents of unreacted amine reactant used in step (b), and maintaining said reaction zone at a temperature and for a time sufficient to permit reaction of said excess amine reactant and said scavenger;
e) separating the solid supported scavenger from each reaction zone of step (d) and recovering each substantially purified secondary amine library compound.

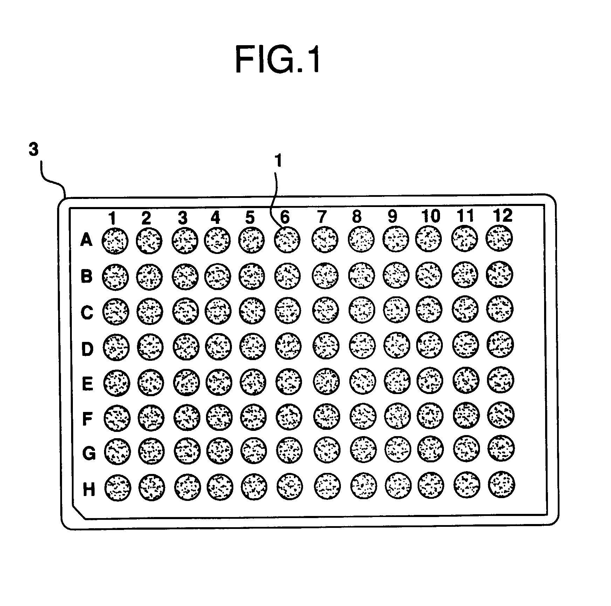

The "adding to each reaction zone" requirement for the amine and carbonyl fuctional reactant in steps (a) and (b) means that different amines and carbonyl compounds may be added to each reaction zone in the library forming process, if desired. In one embodiment of the process of the invention, each combination of amine and carbonyl compound added to each library reaction zone (e.g., wells of a wellplate) is different. Thus, the same amine may be added to each row of a wellplate apparatus (as per Fig. 1) and the same carbonyl reactant may be added to the same column of a wellplate apparatus to give a different combination of reactants in each well (viz., reaction zone) that will expectantly yield a different library compound. Alternatively, where it is desirable to have replicate samples, the same combination of amine and carbonyl compound may be added to different reaction zones.

### V. Detail of Operation for the Reductive Amination Process - Step (a):

The reductive amination process is conducted using in step (a) a carbonyl functional reactant represented by the formula: 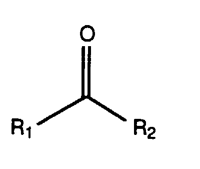 wherein R₁ and R₂ are not both hydrogen and are the same or different and are preferably selected from C₁-C₁₀ alkyl, C₂-C₁₀ alkenyl, C₂-C₁₀ alkynyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, -(CH₂)ₘ-O-(C₁-C₁₀ alkyl), heterocyclic radical, substituted heterocyclic radical, aryl, and substituted aryl.

The carbonyl functional reactant is preferably selected from aliphatic, aromatic, and heterocyclic carbonyl functional compounds having a molecular weight of from 50 to 600.

Examples of carbonyl functional reactants suitable for use in the process of the invention are the following:
Aldehyde reagents --
   2-fluorenecarboxaldehyde
   n-methylpyrrole-2-carboxaldehyde
   furfural
   5-nitro-2-furaldehyde
   5-methylfurfural
   5-acetoxymethyl-2-furaldehyde
   5-hydroxymethyl-2-furaldehyde
   benzaldehyde
   2-bromobenzaldehyde
   6-bromoveratraldehyde
   2-fluorobenzaldehyde
   pentafluorobenzaldehyde
   2-chlorobenzaldehyde
   2,4-dichlorobenzaldehyde
   2-chloro-6-fluorobenzaldehyde
   2,6-dichlorobenzaldehyde
   o-anisaldehyde
   2,3-dimethoxybenzaldehyde
   2,3,4-trimethoxybenzaldehyde
   2,4-dimethoxybenzaldehyde
   2,4,5-trimethoxybenzaldehyde
   2,4,6-trimethoxybenzaldehyde
   2,5-dimethoxybenzaldehyde
   2-ethoxybenzaldehyde
   salicylaldehyde
   3,5-dibromosalicylaldehyde
   3-fluorosalicylaldehyde
   3,5-dichlorosalicylaldehyde
   3,5-diiodosalicylaldehyde
   o-vanillin
   3-ethoxysalicylaldehyde
   2,3-dihydroxybenzaldehyde
   2,3,4-trihydroxybenzaldehyde
   4-(diethylamino)salicylaldehyde
   2-hydroxy-4-methoxybenzaldehyde
   4,6-dimethoxy-2-hydroxybenzaldehyde
   2,4,6-trihydroxybenzaldehyde
   5-bromosalicylaldehyde
   5-chlorosalicylaldehyde
   2-hydroxy-5-methoxybenzaldehyde
   2,5-dihydroxybenzaldehyde
   2-carboxybenzaldehyde
   2-(trifluoromethyl)benzaldehyde
   o-tolualdehyde
   2,3-dimethyl-p-anisaldehyde
   2,4-dimethylbenzaldehyde
   mesitaldehyde
   2,5-dimethylbenzaldehyde
   2,5-dimethyl-p-anisaldehyde
   3-cyanobenzaldehyde
   3-bromobenzaldehyde
   3-bromo-4,5-dimethoxybenzaldehyde
   5-bromo-2-methoxybenzaldehyde
   3-fluorobenzaldehyde
   3-fluoro-p-anisaldehyde
   3-chlorobenzaldehyde
   3,4-dichlorobenzaldehyde
   3,5-dichlorobenzaldehyde
   3-phenoxybenzaldehyde
   3-(3,4-dichlorophenoxy)benzaldehyde
   3-(3,5-dichlorophenoxy)benzaldehyde
   3-(3-(trifluoromethyl)phenoxy)benzaldehyde
   3-(4-chlorophenoxy)benzaldehyde
   3-(4-methoxyphenoxy)benzaldehyde
   3-(4-tert-butylphenoxy)benzaldehyde
   3-(4-methylphenoxy)benzaldehyde
   m-anisaldehyde
   4-acetoxy-3-methoxybenzaldehyde
   3,4-dimethoxybenzaldehyde
   3,4,5-trimethoxybenzaldehyde
   4-benzyloxy-3-methoxybenzaldehyde
   3,5-dimethoxybenzaldehyde
   3-benzyloxybenzaldehyde
   3-hydroxybenzaldehyde
   3-hydroxy-4-methoxybenzaldehyde
   3,4-dihydroxybenzaldehyde
   3,4,5-trihydroxy benzaldehyde
   3-(trifluoromethyl)benzaldehyde
   m-tolualdehyde
   3-methyl-p-anisaldehyde
   4-cyanobenzaldehyde
   4-bromobenzaldehyde
   4-fluorobenzaldehyde
   4-chlorobenzaldehyde
   4-acetamidobenzaldehyde
   4-dimethylaminobenzaldehyde
   4-diethylaminobenzaldehyde
   4-phenoxybenzaldehyde
   4-acetoxybenzaldehyde
   p-anisaldehyde
   3-benzyloxy-4-methoxybenzaldehyde
   4-benzyloxybenzaldehyde
   4-ethoxybenzaldehyde
   4-n-butoxybenzaldehyde
   1-naphthaldehyde
   2-methoxy-1-naphthaldehyde
   2-hydroxy-1-naphthaldehyde
   4-methoxy-1-naphthaldehyde
   2-naphthaldehyde
   1-pyrenecarboxaldehyde
   3,4-dibenzyloxybenzaldehyde
   n-ethyl-3-carbazolecarboxaldehyde
   2-methyl-9-acridinecarboxaldehyde
   pyrrole-2-carboxaldehyde
   2-thiophenecarboxaldehyde
   3-methylthiophene-2-carboxaldehyde
   4-bromothiophene-2-aldehyde
   5-bromo-2-thiophenecarboxaldehyde
   5-nitrothiophene-2-carboxaldehyde
   5-methyl-2-thiophenecarboxaldehyde
   3-thiophenecarboxaldehyde
   indole-3-carboxaldehyde
   5-methoxyindole-3-carboxaldehyde
   piperonal
   6-nitropiperonal
   2-pyridinecarboxaldehyde
   6-methyl-2-pyridinecarboxaldehyde
   3-pyridinecarboxaldehyde
   4-pyridinecarboxaldehyde
   3-quinolinecarboxaldehyde
   4-quinolinecarboxaldehyde
   4-hydroxybenzaldehyde
   5-bromovanillin
   5-iodovanillin
   vanillin
   syringaldehyde
   3-ethoxy-4-hydroxybenzaldehyde
   3,5-dimethyl-4-hydroxybenzaldehyde
   4-biphenylcarboxaldehyde
   4-(methylthio)benzaldehyde
   methyl 4-formylbenzoate
   4-carboxybenzaldehyde
   4-trifluoromethylbenzaldehyde
   4-isopropylbenzaldehyde
   p-tolualdehyde
   4-ethylbenzaldehyde
   4-chloro-3-nitrobenzaldehyde
   3,5-dinitro-2-hydroxybenzaldehyde
   3-hydroxy-4-nitrobenzaldehyde
   4-hydroxy-3-nitrobenzaldehyde
   5-nitrovanillin
   2-nitrobenzaldehyde
   2,6-dinitrobenzaldehyde
   6-nitroveratraldehyde
   3-methoxy-2-nitrobenzaldehyde
   2-chloro-6-nitrobenzaldehyde
   3-nitrobenzaldehyde
   5-chloro-2-nitrobenzaldehyde
   2-chloro-5-nitrobenzaldehyde
   5-hydroxy-2-nitrobenzaldehyde
   5-nitrosalicylaldehyde
   4-nitrobenzaldehyde
   1,4-benzodioxan-6-carboxaldehyde
   2,3-dichlorobenzaldehyde
   3-ethoxy-4-methoxybenzaldehyde
   3,5-bis(trifluoromethyl)benzaldehyde
   2,3,6-trichlorobenzaldehyde
   terephthalaldehyde monodiethylacetal
   2,3-difluorobenzaldehyde
   2,6-difluorobenzaldehyde
   2,4-difluorobenzaldehyde
   2,5-difluorobenzaldehyde
   3,4-difluorobenzaldehyde
   3,5-difluorobenzaldehyde
   4-dimethylamino-1-naphthaldehyde
   3-furaldehyde
   3,4-dimethoxy-5-hydroxybenzaldehyde
   2,3,5-trichlorobenzaldehyde
   2,6-dimethoxybenzaldehyde
   5-bromo-2,4-dimethoxybenzaldehyde
   2,4-dimethoxy-3-methylbenzaldehyde
   4-stilbenecarboxaldehyde
   4-(3-dimethylaminopropoxy)benzaldehyde
   2,4-dihydroxybenzaldehyde
   3-chloro-4-fluorobenzaldehyde
   2-methylindole-3-carboxaldehyde
   4-hydroxy-3-methylbenzaldehyde
   2-(diphenylphosphino)benzaldehyde
   2,4-dinitrobenzaldehyde
   4-n-propoxybenzaldehyde
   5-bromo-2-hydroxy-3-methoxybenzaldehyde
   3-bromo-4-methoxybenzaldehyde
   4-acetoxy-3,5-dimethoxybenzaldehyde
   3,5-dihydroxybenzaldehyde
   3-methoxy-4-(4-nitrobenzyloxy)benzaldehyde
   2,3-(methylenedioxy)benzaldehyde
   2-hydroxy-3-methoxy-5-nitrobenzaldehyde
   2-cyanobenzaldehyde
   5-ethyl-2-furaldehyde
   4-tert-butylbenzaldehyde
   3-tetrafluoroethoxybenzaldehyde
   3-carboxybenzaldehyde
   4-(trifluoromethoxy)benzaldehyde
   3-bromo-4-fluorobenzaldehyde
   3-(trifluoromethoxy)benzaldehyde
   2-chloro-4-fluorobenzaldehyde
   5-(3-nitrophenyl)furfural
   2-chloro-4-hydroxybenzaldehyde
   2,3,4-trifluorobenzaldehyde
   2-fluoro-3-(trifluoromethyl)benzaldehyde
   2-fluoro-6-(trifluoromethyl)benzaldehyde
   4-fluoro-2-(trifluoromethyl)benzaldehyde
   2-quinolinecarboxaldehyde
   4-(dibutylamino)benzaldehyde
   5-(trifluoromethoxy)salicylaldehyde
   3-fluoro-2-methylbenzaldehyde
   3,5-dibenzyloxybenzaldehyde
   5-(4-nitrophenyl)furfural
   2-chloro-3-quinolinecarboxaldehyde
   5-bromo-3-nitrosalicylaldehyde
   2-chloro-5-(trifluoromethyl)benzaldehyde
   5-bromo-2-furaldehyde
   2,3,5,6-tetrafluorobenzaldehyde
   4-methyl-5-imidazolecarboxaldehyde
   2-benzyloxy-4,5-dimethoxybenzaldehyde
   3,5-di-tert-butyl-2-hydroxybenzaldehyde
   2,4-diethoxy-m-tolualdehyde
   4-tert-pentylbenzaldehyde
Ketone reagents --
   2-fluorophenylacetone
   4-fluorophenylacetone
   3-trifluoromethylphenylacetone
   1,1,1-trifluoro-2,4-pentanedione
   cyclotridecanone
   cyclobutanone
   cyclopentanone
   3-methylcyclopentanone
   cyclohexanone
   3-methylcyclohexanone
   3,3,5,5-tetramethylcyclohexanone
   4-phenylcyclohexanone
   4-tert-butylcyclohexanone
   4-methylcyclohexanone
   4-ethylcyclohexanone
   beta-tetralone
   6-methoxy-2-tetralone
   7-methoxy-2-tetralone
   2-decalone
   cyclooctanone
   cyclononanone
   4-(4-hydroxyphenyl)-2-butanone
   3-acetyl-1-propanol
   cyclodecanone
   cycloundecanone
   cyclododecanone
   2 -indanone
   cycloheptanone
   4-hydroxy-4-methyl-2-pentanone
   1,3-diphenylacetone
   1-benzyl-3-pyrrolidinone
   tetrahydrothiophen-3-one
   tropinone
   n-carbethoxy-4-piperidone
   n-benzoyl-4-piperidone
   1-acetyl-4-piperidone
   1-methyl-4-piperidone
   1-benzyl-4-piperidone
   1-(beta-phenylethyl)-4-piperidone
   tetrahydro-4h-pyran-4-one
   tetrahydrothiopyran-4-one
   4-(4-acetoxyphenyl)-2-butanone
   acetone
   (dimethylamino)acetone
   phenoxyacetone
   methoxyacetone
   2-methoxyphenylacetone
   3-methoxyphenylacetone
   3,4-dimethoxyphenylacetone
   4-methoxyphenylacetone
   benzylacetone
   4-(4-methoxyphenyl)-2-butanone
   5-hexen-2-one
   4,4-dimethyl-2-pentanone
   6-methyl-5-hepten-2-one
   geranylacetone
   2,6-dimethyl-4-heptanone
   5-methyl-2-hexanone
   2-tridecanone
   1-diethylamino-3-butanone
   5-diethylamino-2-pentanone
   ethyl levulinate
   diethyl 4-oxopimelate
   ethyl 4-acetylbutyrate
   1-phenyl-2-butanone
   3 -pentanone
   5-methyl-3-heptanone
   2 -pentanone
   3 -hexanone
   4-heptanone
   butyl levulinate
   2-hexanone
   3 -heptanone
   5-nonanone
   2-heptanone
   3 -octanone
   6-undecanone
   2 -octanone
   3 -nonanone
   4-decanone
   7-tridecanone
   2 -nonanone
   3 -decanone
   2 -decanone
   2 -undecanone
   6,7-dimethoxy-2-tetralone
   5-cholesten-3-one
   4-acetoxy-2-butanone
   1-hydroxy-2-butanone
   1-propyl-4-piperidone
   5alpha-androst-16-en-3-one
   5-ketohexanenitrile
   methyl ethyl ketone
   methyl 7-oxooctadecanoate
   methyl 12-oxooctadecanoate
   8-cyclohexadecen-1-one
   ethyl 4-oxocyclohexanecarboxylate
   1-ethyl-4-piperidone
   5-methyl-5-hexen-2-one
   3,3,5-trimethylcyclohexanone
   2-hexadecanone
   (r)-(+)-3-methylcyclohexanone
   (r)-(+)-3-methylcyclopentanone
   5-alpha-cholestan-3-one
   nerylacetone
   methyl 3-oxo-6-octenoate
   phthalimidoacetone
   4-hydroxy-3-methoxyphenylacetone
   zearalenone
   methyl 5-oxooctadecanoate
   (+/-)-exo-6-hydroxytropinone
   1-benzyl-3-piperidone hydrochloride hydrate

### VI. Detail of Operation for the Reductive Amination Process - Step (b):

The reductive amination process is preferably conducted using in step (b) using a soluble primary amine reactant;

R₃-NH₂

where R₃ is preferably C₁-C₁₀ alkyl, C₂-C₁₀ alkenyl, C₂-C₁₀ alkynyl, C₁-C₁₀ alkoxy, C₇-C₁₂ aralkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₁-C₆ alkylsulfinyl, C₁-C₁₀ alkylsulfonyl, -(CH₂)ₘ-O-(C₁-C₁₀ alkyl), aryl, substituted aryl, substituted alkoxy, amidino, fluoroalkyl, aryloxyalkyl, substituted heterocyclic radical, and nitroalkyl; where m is from 1 to 8. Preferred non-interfering radicals are C₁-C₁₀ alkyl, C₂-C₁₀ alkenyl, C₁-C₁₀ alkoxy, C₇-C₁₂ aralkyl, C₇-C₁₂ alkaryl, C₃-C₁₀ cycloalkyl, C₃-C₁₀ cycloalkenyl, and -(CH₂)ₘ-O-(C₁-C₁₀ alkyl),

The primary amine reactant;

R₃-NH₂

of step (b) is preferably selected from aliphatic primary amines having a molecular weight of from 50 to 600.

Examples of suitable amine reactants for use in the process of the invention are the following:
Primary Amine Reagents --
   aniline
   cyclopropylamine
   cyclobutylamine
   (-)-cis-myrtanylamine
   cyclopentylamine
   cyclohexylamine
   2-methylcyclohexylamine
   2,3-dimethylcyclohexylamine
   4-methylcyclohexylamine
   (aminomethyl)cyclohexane
   3-aminomethyl-3,5,5-trimethylcyclohexanol
   1,2,3,4-tetrahydro-1-naphthylamine
   cyclooctylamine
   l-tyrosine methyl ester
   2-(2-aminoethyl)-1-methylpyrrolidine
   n-(2-aminoethyl)pyrrolidine
   n-(3'-aminopropyl)-2-pyrrolidinone
   furfurylamine
   cyclododecylamine
   1-aminoindan
   dl-1-(1-naphthyl)ethylamine
   1-naphthalenemethylamine
   cycloheptylamine
   (1s,2s)-(+)-2-amino-1-phenyl-1,3-propanediol
   dl-2-amino-3-methyl-1-butanol
   l-isoleucinol
   l-phenylalaninol
   dl-4-chlorophenylalaninol
   d-(-)-leucinol
   l-methioninol
   histamine
   tetrahydrofurfurylamine
   dl-alpha-methyltryptamine
   tryptamine
   5-methoxytryptamine
   6-methoxytryptamine
   piperonylamine
   n-(2-aminoethyl)morpholine
   n-(3-aminopropyl)morpholine
   2-(2-aminoethylamino)-5-nitropyridine
   2-(aminomethyl)pyridine
   2-(2-aminoethyl)pyridine
   3-(aminomethyl)pyridine
   4-(aminomethyl)pyridine
   ethyl 4-amino-1-piperidinecarboxylate
   4-amino-1-benzylpiperidine
   1-(2-aminoethyl)piperidine
   1-(3-aminopropyl)-2-pipecoline
   1,2-diamino-2-methylpropane
   benzhydrylamine
   d-(-)-alpha-phenylglycinol
   1,2-diphenylethylamine
   dl-1-phenylethylamine
   (-)-norephedrine
   1,2-dimethylpropylamine
   isopropylamine
   2-methoxyisopropylamine
   dl-2-amino-1-propanol
   ethyl-3-aminobutyrate
   1,3-dimethylbutylamine
   3-amino-1-phenylbutane
   2-amino-5-diethylaminopentane
   1,5-dimethylhexylamine
   sec-butylamine
   (+/-)-2-amino-1-butanol
   3 -aminopentane
   2 -aminopentane
   3 -aminoheptane
   2 -aminoheptane
   2 -aminooctane
   benzylamine
   2-fluorobenzylamine
   2-chlorobenzylamine
   2,4-dichlorobenzylamine
   2-methoxybenzylamine
   2-ethoxybenzylamine
   2-methylbenzylamine
   3-fluorobenzylamine
   3,4-dichlorobenzylamine
   3,4-dimethoxybenzylamine
   3-(trifluoromethyl)benzylamine
   3-methylbenzylamine
   4-fluorobenzylamine
   4-chlorobenzylamine
   4-methoxybenzylamine
   4-methylbenzylamine
   2,2,2-trifluoroethylamine
   2-amino-1-phenylethanol
   1-amino-2-propanol
   3-amino-1,2-propanediol
   2,2-diphenylethylamine
   beta-methylphenethylamine
   isobutylamine
   2-methylbutylamine
   2-ethylhexylamine
   n-decylamine
   n-undecylamine
   dodecylamine
   tridecylamine
   1-tetradecylamine
   hexadecylamine
   octadecylamine
   ethylamine
   2-(2-aminoethylamino)ethanol
   2-methoxyethylamine
   2-(2-aminoethoxy)ethanol
   ethanolamine
   phenethylamine
   2-(2-chlorophenyl)ethylamine
   2-(2-methoxyphenyl)ethylamine
   3-methoxyphenethylamine
   2-(3,4-dimethoxyphenyl)ethylamine
   4-bromophenethylamine
   2-(4-chlorophenyl)ethylamine
   2-(4-methoxyphenyl)ethylamine
   tyramine
   2-(4-aminophenyl)ethylamine
   2-(p-tolyl)ethylamine
   taurine
   propargylamine
   allylamine
   3,3-dimethylbutylamine
   3,3-diphenylpropylamine
   isoamylamine
   propylamine
   3-dimethylaminopropylamine
   3-diethylaminopropylamine
   3-(di-n-butylamino)propylamine
   3-isopropoxypropylamine
   3-ethoxypropylamine
   3-amino-1-propanol
   3-phenylpropylamine
   4-amino-1-butanol
   4-phenylbutylamine
   n-amylamine
   5-amino-1-pentanol
   hexylamine
   6-amino-1-hexanol
   n-heptylamine
   n-octylamine
   n-nonylamine
   dl-2-amino-1-pentanol
   dl-2-amino-1-hexanol
   1-(3-aminopropyl)imidazole
   3,5-bis(trifluoromethyl)benzylamine
   2,4-difluorobenzylamine
   2,5-difluorobenzylamine
   2,6-difluorobenzylamine
   3,4-difluorobenzylamine
   4-(trifluoromethyl)benzylamine
   2-(trifluoromethyl)benzylamine
   4-(2-aminoethyl)benzenesulfonamide
   n-(4-aminobutyl)-n-ethylisoluminol
   n-butylamine
   2-(1-cyclohexenyl)ethylamine
   3-methoxypropylamine
   3,4,5-trimethoxybenzylamine
   3-butoxypropylamine
   aminomethylcyclopropane
   pentadecylamine
   4-(2,4-di-tert-amylphenoxy)butylamine
   3-chlorobenzylamine
   4-fluoro-alpha-methylbenzylamine
   (r)-(+)-bornylamine
   n,n-di-n-butylethylenediamine
   (r)-(-)-1-cyclohexylethylamine
   n,n,2,2-tetramethyl-1,3-propanediamine
   l-phenylalanine beta-naphthyl-amide
   2-(3-chlorophenyl)ethylamine
   2-amino-1,3-propanediol
   2-(2-thienyl)ethylamine
   2,3-dimethoxybenzylamine
   3,5-dimethoxybenzylamine
   2,4-dichlorophenethylamine
   2,5-dimethoxyphenethylamine
   3-fluoro-5-(trifluoromethyl)benzylamine
   4-(trifluoromethoxy)benzylamine
   l-leucinol
   l-leucine-4-nitroanilide
   (r)-(+)-1-(1-naphthyl)ethylamine
   (s)-(-)-1-(1-naphthyl)ethylamine
   l-valinol
   d-valinol
   d-phenylalaninol
   l-(+)-alpha-phenylglycinol
   d-(+)-alpha-methylbenzylamine
   l(-)-alpha-methylbenzylamine
   (1s,2r)-(+)-phenyl-propanolamine
   (s)-(+)-2-amino-1-propanol
   d-alaninol
   (r)-(-)-sec-butylamine
   (s)-(+)-sec-butylamine
   (s)-(+)-2-amino-1-butanol
   (r)-(-)-2-amino-1-butanol
   (r)-(-)-1-amino-2-propanol
   (s)-(+)-1-amino-2-propanol
   (s)-(-)-2-methylbutylamine
   (s)-(+)-1-cyclohexylethylamine
   oleylamine
   1-adamantanemethylamine
   (1s,2r)-(+)-2-amino-1,2-diphenylethanol
   (1r,2s)-(-)-2-amino-1,2-diphenylethanol
   s-benzyl-l-cysteinol
   2-(2-(aminomethyl)phenylthio)benzyl alcohol
   3-fluorophenethylamine
   2-aminobenzylamine
   2-fluorophenethylamine
   4-aminobenzylamine
   d-glucamine
   (+/-)-2,5-dihydro-2,5-dimethoxyfurfurylamine
   (s)-(+)-tetrahydrofurfurylamine
   4-fluorophenethylamine
   (1s,2s)-(+)-thiomicamine
   (-)-3,4-dihydroxynorephedrine
   (r)-(+)-1-(p-tolyl)ethylamine
   (s)-(-)-1-(p-tolyl)ethylamine
   (s)-(-)-2-amino-1,1-diphenyl-1-propanol
   (+/-)-exo-2-aminonorbornane
   (s)-(+)-2-(aminomethyl)pyrrolidine
   3-amino-1-propanol vinyl ether
   geranylamine
   4-(hexadecylamino)benzylamine
   (1r,2r,3r,5s)-(-)-isopinocampheylamine
   (1s,2s,3s,5r)-(+)-isopinocampheylamine
   n1-isopropyldiethylenetriamine
   (s)-tert-leucinol
   (r)-(-)-tetrahydrofurfurylamine
   dehydroabietylamine
   2-bromo-4,5-dimethoxyphenethylamine
   (1s,2r)-(-)-cis-1-amino-2-indanol
   (1r,2s)-(+)-cis-1-amino-2-indanol.

Additional primary amines suitable for the process of the invention are those represented by the following formulae: 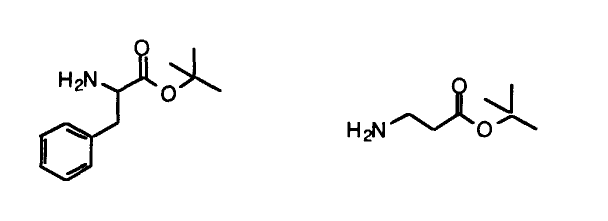 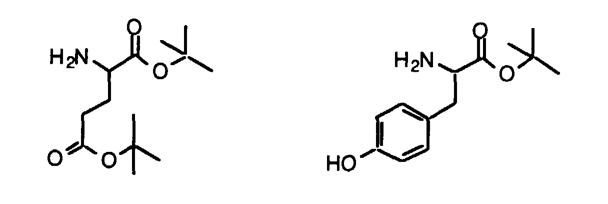 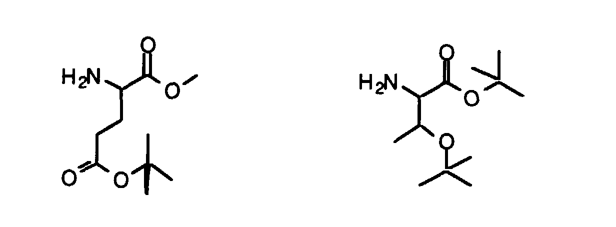 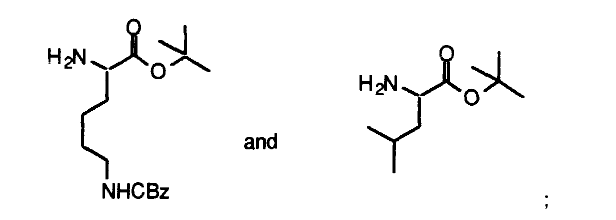 where CBz is benzyloxycarbonyl.

### VII. Detail of Operation for the Reductive Amination Process - Step (c):

The reducing agent used in step (c) of the process may be any conventional reagent and attendant methods used for conducting reduction reactions; inclusive of H₂/Pd, H₂ via high pressure hydrogenation, lithium aluminum hydride, sodium borohydride, borohydride functional polymer, and cyanoborohydride functional polymer.

The borohydride functional polymer is a preferred reagent for use in the process of this invention. It is insoluble in the reaction media and may be conveniently removed in step (e) of this process by mechanical techniques (e.g., filtration). Cyanoborohydride functional polymer reducing agent may be prepared according to the procedure Goudgaon, et. al. - Ref (d) supra. Commercially prepared borohydride functional polymer may be purchased as Amberlite IRA 400 borohydride resin from Aldrich Chemicals.

### VIII. Detail of Operation for the Reductive Amination Process - Step (d):

The liquid reaction medium insoluble amine reactive scavenger added to each reaction zone of step (c) is represented by the formula; 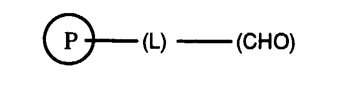 wherein; 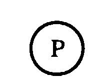 is a solid-support insoluble in the liquid medium of the reaction zone, -(L)- is a divalent linking group, and (CHO) is an aldehyde functional substituent.

Examples of organic solid supports are polymers such as polystyrene/divinylbenzene copolymer, polyacrylamide, cellulose and polystyrene. Examples of inorganic solid supports are silica gel, alumina, and controlled pore glass.

The group, -(L)-, is a linking group between the aldehyde radical and the solid support and may be selected from a bond, or any divalent group. Preferably -(L)- is a divalent linking group of 1 to 40 atoms. Useful linking groups are selected from the following:
(bond),

-O-(CH₂)ₓ-

-S-(CH₂)ₓ-

-CH₂-(CH₂)₀₋₁₀-

where x is an integer from 2 to 10.

The aldehyde substituent on the scavenger readily reacts with the excess primary amine reagent used in step (b) of the process to covalently bind said excess reagent to the solid support and permit its simple removal as a solid phase. The effective available aldehyde content of the solid supported scavenger may be readily determined by conventional chemical analysis techniques (e.g., elemental analysis).

The amount of solid supported amine scavenger, 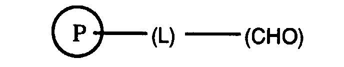 used in step (d) is based on the scavenger's available aldehyde functionality. The scavenger is added in at least an amount equal to the theoretical excess equivalents of unreacted primary amine reactant (viz., at least 0.1 equivalents used in step (b). For example, if 1.25 mmol. of benzylamine (constituting a 0.25 mmol. excess) were used in step (b), then at least 0.25 mmol. of an aldehyde functional polymer would be added in step (d). Preferably, the solid supported scavenger is used in an amount that is from 1.25 to 5 times the theoretical excess equivalents of unreacted amine reagent.

The reaction zone is maintained at a temperature for a time sufficient to permit reaction of said excess primary amine reactant and said amine reactive scavenger.

### IX. Detail of Operation for the Reductive Amination Process - Step (e):

The final step in the secondary amine library forming process of the invention is purification of the library compounds by separating the reacted and unreacted solid supported scavenger from the reaction medium of step (d) and recovering a solution of each substantially purified secondary amine library compound.

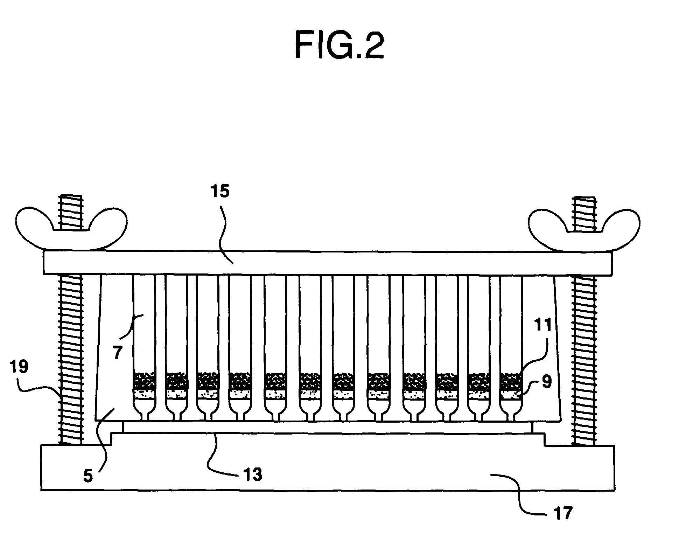

The separation of the solid supported scavenger from the library compound dissolved in the solvent phase of the reaction may be done by any conventional chemical or physical method. Preferred are physical methods which are applicable to all members of a diverse library. Such methods include; (i) filtration, (ii) centrifugation, (iii) decantation, and (iv) washing. Filtration is a particularly preferred form of purification. It is practiced by transporting each solution of library compound through a filter medium which retains the scavenger and transfers the solution phase into a separate vessel. An apparatus using filtration is depicted in Fig. 2, infra.

The purification last step of the process may optionally be supplemented by a solvent removal step in which the solute library compound is removed from its solvent by conventional processes known in the art; such as evaporation, distillation, freeze drying, salting out, solvent extraction, and etc.

### X. Other Details of the Reductive Amination Secondary Amine Library making Process:

Reaction Medium - The reaction medium may be any liquid which has the following characteristics:
(1) the primary amine and carbonyl functional reactants are capable of forming a reaction product which is substantially soluble in the reaction medium, and
(2) the solid supported scavenger, both in unreacted and reacted form, is substantially insoluble in the reaction medium.

Typical reaction media useful in the processes of the invention are methanol, ethanol, methylene chloride, acetonitrile. Preferred reaction media is methanol mixed with methylene chloride.

The Reaction Zone - the process of the invention may be carried out in any vessel capable of holding the liquid reaction medium and having inlet and outlet means. Preferably the process of the invention is carried out in containers adaptable to parallel array syntheses. Most preferably, the secondary amine library is formed in standard wellplates, such as the 96 well wellplate illustrated in Fig. 1 and/or the wellplate apparatus illustrated in Fig. 2. Each well may be filled by multiple delivery apparatus, automated or robotic apparatus, any of which may be either manually or computer controlled.

The diverse secondary amine library of this invention may take the form of a plurality of wellplates, each wellplate having wells containing a separate reaction product (library compound). In such cases, the library compounds are conveniently identified by their wellplate number and "x" column and "y" wellplate row coordinates.

A preferred technique for practicing the process of the invention is parallel array synthesis. With parallel array synthesis individual reaction products are prepared in each of multiple reaction zones. The amount of secondary amine and carbonyl reactants introduced into each reaction zone will depend on the desired amount of each library compound that is needed for conducting biological assays, archival storage and other related needs. Typically, the desired amount of individual reaction product is from 1 microgram to 50 milligrams.

### Proportions of reactants, reaction conditions:

The amount of carbonyl functional reactant in each reaction zone is represented by the symbol "(n)", where (n) represents the equivalents of aldehyde or ketone.

In the diverse secondary amine library making process described herein the primary amine reactant is the reactant used in excess. The method of the invention contemplates solution phase reactions where a stoichiometric excess of primary amine reactant is used. The amount of primary amine used to insure an excess is defined as at least 1.1(n) and preferably a larger excess in the range of from 1.25(n) to 5(n), where the variable (n) is as previously defined. The stoichiometry of the reaction and calculation of equivalent weights of reagents may be done by reference to Scheme 1, infra. The 1.1 multiplier is used to insure at least a 10% stoichiometric excess of the primary amine to drive the reaction to completion, thereby removing the aldehyde reactant from each reaction zone used to create the secondary amine library. Thus, for example, if 1.5(n) - a 50% excess - of the primary amine reactant is desired, then 35 mg. of benzaldehyde would be used in step (a) of the process and 80 mg. of tryptamine would be used in step (b) of the process.

The reaction zone is maintained at a temperature and for a time sufficient to permit reaction of the carbonyl compound and primary amine reactants, that is, to complete consumption of the aldehyde and form an imine. Thereafter, the reducing agent is added.

The time, temperature, and pressure of the combinatorial reaction zones used for the creation of library compounds are not critical aspects of the invention. Reaction times for imine formation are generally from 0.1 seconds to 24 hours, with times of 1 second to 60 minutes being most often used. The reduction step of the process is typically from 1 to 6 hours. The temperature of the reaction may be any temperature between the freezing point and the boiling point of the liquid reaction medium, but is generally between -10°C and +60°C, with 10°C to 40°C being preferred and ambient temperatures (about 20°C-30°C) being most preferred. The reactions may be conducted at subatmospheric pressure or superatmospheric pressure (viz., 60Kg./m² - 21000 Kg./m² absolute), but ambient atmospheric pressure (about 10330 Kg./m², absolute) is most often used.

Endpoint determination - The completion of the reaction between the imine and hydride may be determined by a number of conventional techniques.

Sequence of Operation - The addition of the carbonyl compound and primary amine reactants to the reaction zone may take place in any order. For example, the primary amine reactant may be initially added to the reaction zone followed by addition of the carbonyl compound reactant, or vice versa. Alternatively, the primary amine and carbonyl reactants may be simultaneously charged to each reaction zone.

The reaction zone is maintained at a temperature for a time sufficient to permit reaction of said excess primary amine reactant and said scavenger. Typically, the reaction requires only seconds but the selection of reaction conditions that may be used is the same as set out in the preceding section.

### XI. Reductive Amination Process - Reaction Scheme:

To make secondary amines reductive amination of primary amines may be done with an aldehyde (or ketone). For combinatorial processes an excess of primary amine relative to aldehyde (or ketone) is used. After preformation of the corresponding imine adduct (in methanol), reduction was performed using polymer-supported borohydride. Excess primary amine was readily separated from the desired secondary amine product by selective imine formation using a polymer-supported aldehyde. Filtration and evaporation provided secondary amines with only trace quantites of impurities. If desired, the resin bound borohydride and resin bound aldehyde can be added simultaneously; polymer site isolation and the relative kinetics of imine formation on- and off-polymer allow for this convenient modification in the experimental procedure.

An illustrative reaction scheme for the reductive amination process for preparing libraries of secondary amines is shown in Scheme 1, below:

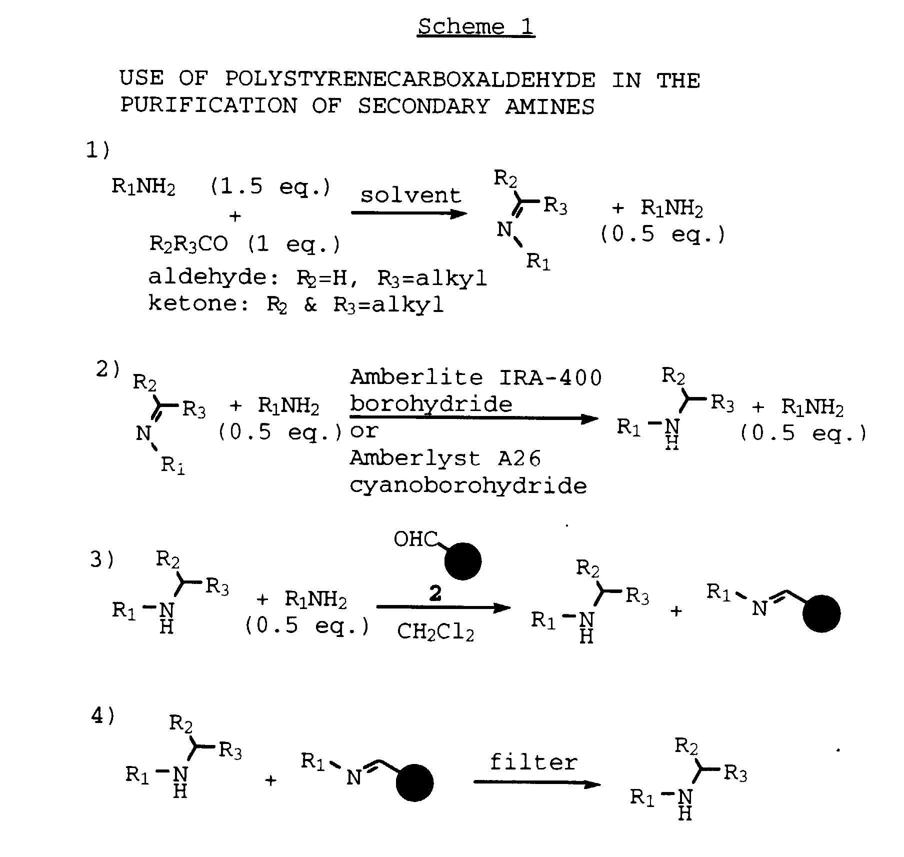

In the use of polystyrenecarboxaldehyde **2** as a scavenger for primary amines in the synthesis of secondary amines (above), a ketone or aldehyde is combined with a 1.5 fold excess of a primary aliphatic amine in methanol as solvent, or a 1.5 fold excess of an aromatic amine with 10% acetic acid in dichloroethane as solvent (Step 1). After imine formation is complete (typically one hour for aldehydes, 24 hours for ketones), a resin bound reducing agent is added to the solution (Amberlite IRA-400 borohydride for neutral solutions, Amberlyst A26 cyanoborohydride for acidic solutions), and the solutions are shaken until reduction is complete (Step 2). To this mixture is added an excess (relative to the remaining primary amine) of polystyrene benzaldehyde **2** (Step 3), which reacts with primary amine present to form a resin bound imine species. In the final step, the mixture of product secondary amine and resin is filtered through a cotton plug and the resin, retained by the plug, is rinsed free of any product by washing with additional methanol (Step 4). Note: if an aliphatic amine hydrochloride salt is used in Step 1, it should be free-based by addition of excess resin bound tertiary amine base to promote imine formation.

### Other Utilities - Secondary Amines:

Derivatives of secondary amines (capable of being prepared by the method of this invention) have utility in a number of technological areas, as follows:
A) Various secondary alkyl amines such as di-n-butylamine are catalysts for making polyphenylene ethers.
B) Fatty acid amines generally are useful as emulsifiers and foaming agents.
C) Secondary amines generally are useful as catalysts for the preparation of substituted pyridines (U.S. Pat. No. 5,438,143).
D) Corrosion inhibitors, adhesion promoters, herbicides, and rubber accelerators are prepared as derivatives of simple secondary amines.

### XII. The wellplate apparatus of the invention:

A wellplate inoculated with novel diverse secondary amine compounds of the invention is itself a new construct or apparatus which has particular utility in an assay kit used to discover lead compounds.
A suitable system of operation and related apparatus are made as follows:
1. Reaction zones are made by drilling 96 holes in the bottom of 96 deepwell titer plates and putting a porous frit in the bottom of each well.
2. The plate is put in a clamp assembly to seal the bottom of the wells.
3. Synthesis is begun by adding reagents (viz., the amine and isocyanate reactants) to their assigned plate coordinates (reaction zone).
4. The plate is capped then tumbled to mix the reagents.
5. Solid supported scavenger is added to each reaction zone after completion of the reaction is shown by thin layer chromatography.
6. After sufficient reaction time the plate is removed from the clamp and the resin washed.
7. The solution containing product is filtered and the solution collected by transfer into another 96 well plate.
8. The reaction products (library compounds) are analyzed by thin layer chromatography.

### Detailed Description of the Drawings

FIG. 1 illustrates the top surface of a wellplate apparatus of the invention. The wellplate (3) is a plastic plate with 96 wells (depressions) capable of holding liquids. When used in the parallel array synthesis individual reaction products are prepared in each well and are labeled by the wellplate coordinates. The shaded circles in the Figure represent wells filled with secondary library compounds prepared by the solution phase combinatorial process of the invention. The compound at location (1), for example, is identified by the alphanumeric coordinate, "A6."

FIG. 2 illustrates a side view of a wellplate apparatus used in the Assay Kit of the invention. The wellplate (5) contains wells (7) with a filter (9) and liquid reaction medium containing scavenger (11). The wells have an outlet at bottom which is sealed by gasket (13) held in place by top cover (15) and bottom cover (17) maintained in position by clamp (19).

### XIII. The Assay Kit of the Invention:

This invention is an assay kit for identification of pharmaceutical lead compounds., comprising biological assay materials and wellplate apparatus. The assay kit comprises as essential parts, (i) wellplate apparatus (containing in its wells the novel urea or thiourea library compounds of the invention), and (ii) biological assay materials.

The wellplate apparatus in the kit may comprise a set of wellplate apparatus such as illustrated in Fig. 1. The urea or thiourea library compounds contained in each wellplate are prepared by either the urea process or the thiourea library processes taught herein. Preferably the wellplate apparatus has the form of a standard 96 well microtiter plate.

The assay kit also contains biological assay materials These biological assay materials are generally in vitro tests known to be predictive of success for an associated disease state. Illustrative of biological assay materials useful in the kit of this invention are those required to conduct the following assays:
In vitro assays:
   Enzymatic Inhibition
   Receptor-ligand binding
   Protein-protein Interaction
   Protein-DNA Interaction
Cell-based, Functional assays:
   Transcriptional Regulation
   Signal Transduction/ Second Messenger
   Viral Infectivity
Add, Incubate, & Read assays:
   Scintillation Proximity Assays
      Angiotensin II SPA receptor binding assay
      Endothelin converting enzyme[¹²⁵I] SPA assay
      HIV proteinase [¹²⁵I] SPA enzyme assay
      Cholesteryl ester transfer protein (CETP) [³H] SPA assay
   Fluorescence Polarization Assays
   Fluorescence Correlation Spectroscopy
   Colorimetric Biosensors
   Ca²⁺-EGTA Dyes for Cell-based assays
   Reporter Gene Constructs for cell based assays Luciferase, green fluorescent protein, β-lactamase
   Electrical cell impedance sensor assays

### EXAMPLES

Preparation of Polymer Bound Benzaldehyde (**2**): Prepared according to the procedure of Schuerch, with no modifications to the published method. See Frechet, J. M.; Schuerch, C. *J. Am. Chem. Soc.* 1971, 93, 492. Analysis of a typical lot:
Elemental Analysis: C, 90.48; H, 7.57; O, 1.83 (1.1 meq/g loading); Cl, none.
IR (KBr pellet): 1699 cm⁻¹
Commercially available resins:
Amberlite IRA 400 borohydride, Aldrich Chemicals
Poly-DMAP and Polyvinylpyridine, Reilly Scientific

### Reductive Amination Of An Aldehyde And A Primary Amine:

To a 4 mL screw cap glass vial was added 1 mL methanol, 80 mg (0.5 mmol) of tryptamine, and 0.33 mmol (35mg.) of benzaldehyde. The vial was sealed with a teflon backed cap and the solution was then shaken for 1 hour to allow for imine formation, then treated with 1 mL dichloromethane and approximately 250 mg ( 2.5 mmol BH₄⁻/g resin, 0.63 mmol) of Amberlite IRA-400 borohydride resin (Aldrich Chemicals) and approximately 350 mg (1 mmol/g resin, 0.35 mmol) polystyrene-linked benzaldehyde resin in order to scavenge excess primary amine starting material. The slurry was shaken overnight, and then filtered through a cotton plug, and the residual solids were rinsed with methanol. Evaporation under a flow of air, followed by drying for several hours at room temperature in a vacuum oven yielded 69 mg (84%) N-benzyl tryptamine.

The following exemplary carbonyl functional compounds (aldehydes) and primary amines were combined as above to yield 24 additional products:
benzaldehyde (35 mg, 0.33 mmol) and 2-[(2-aminomethyl)phenylthio]benzyl alcohol (122 mg, 0.5 mmol) yielded 88 mg (80%)
   MS (m/e): 336 (M+1)
benzaidehyde (35 mg, 0.33 mmol) and 4-amino-1-benzylpiperidine (93 mg, 0.5 mmol) yielded 79 mg (85%)
   MS (m/e): 281 (M+1)
benzaldehyde (35 mg, 0.33 mmol) and tetrahydrofurfurylamine (51 mg, 0.5 mmol) yielded 44 mg (70%)
   MS (m/e): 192 (M+1)
benzaldehyde (35 mg, 0.33 mmol) and α-methyl benzylamine (60 mg, 0.5 mmol) yielded 57 mg (82%)
   MS (m/e): 212 (M+1)
salicylaldehyde (40 mg, 0.33 mmol) and tryptamine (122 mg, 0.5 mmol) yielded 55 mg (62%)
   MS (m/e): 267 (M+1)
salicylaldehyde (40 mg, 0.33 mmol) and 2-[(2-aminomethyl)phenylthio]benzyl alcohol (122 mg, 0.5 mmol) yielded 78 mg (67%)
   MS (m/e): 352 (M+1)
salicylaldehyde (40 mg, 0.33 mmol) and 4-amino-1-benzylpiperidine (93 mg, 0.5 mmol) yielded 76 mg (78%)
   MS (m/e): 297 (M+1)
salicylaldehyde (40 mg, 0.33 mmol) and tetrahydrofurfurylamine (51 mg, 0.5 mmol) yielded 56 mg (82%)
   MS (m/e): 208 (M+1)
salicylaldehyde (40 mg, 0.33 mmol) and α-methyl benzylamine (60 mg, 0.5 mmol) yielded 51 mg (68%)
   MS (m/e): 228 (M+1)
1-naphthaldehyde (51 mg, 0.33 mmol) and tryptamine (122 mg, 0.5 mmol) yielded 69 mg (70%)
   MS (m/e): 301 (M+1)
1-naphthaldehyde (51 mg, 0.33 mmol) and 2-[(2-aminomethyl)phenylthio]benzyl alcohol (122 mg, 0.5 mmol) yielded 104 mg (82%)
   MS (m/e): 386 (M+1)
1-naphthaldehyde (51 mg, 0.33 mmol) and 4-amino-1-benzylpiperidine (93 mg, 0.5 mmol) yielded 87 mg (80%)
   MS (m/e): 331 (M+1)
1-naphthaldehyde (51 mg, 0.33 mmol) and tetrahydrofurfurylamine (51 mg, 0.5 mmol) yielded 63 mg (79%)
   MS (m/e): 242 (M+1)
1-naphthaldehyde (51 mg, 0.33 mmol) and α-methyl benzylamine (60 mg, 0.5 mmol) yielded 62 mg (72%)
   MS (m/e): 262 (M+1)
2-pyridine carboxaldehyde (35 mg, 0.33 mmol) and tryptamine (122 mg, 0.5 mmol) yielded 74 mg (90%)
   MS (m/e): 252 (M+1)
2-pyridine carboxaldehyde (35 mg, 0.33 mmol) and 2-[(2-aminomethyl)phenylthio]benzyl alcohol (122 mg, 0.5 mmol) yielded 90 mg (81%)
   MS (m/e): 337 (M+1)
2-pyridine carboxaldehyde (35 mg, 0.33 mmol) and 4-amino-l-benzylpiperidine (93 mg, 0.5 mmol) yielded 79 mg (85%)
   MS (m/e): 282 (M+1)
2-pyridine carboxaldehyde (35 mg, 0.33 mmol) and tetrahydrofurfurylamine (51 mg, 0.5 mmol) yielded 24 mg (37%)
   MS (m/e): 193 (M+1)
2-pyridine carboxaldehyde (35 mg, 0.33 mmol) and α-methyl benzylamine (60 mg, 0.5 mmol) yielded 52 mg (74%)
   MS (m/e): 213 (M+1)
4-tolualdehyde (41 mg, 0.33 mmol) and tryptamine (122 mg, 0.5 mmol) yielded 75 mg (86%)
   MS (m/e): 265 (M+1)
4-tolualdehyde (41 mg, 0.33 mmol) and 2-[(2-aminomethyl)phenylthio]benzyl alcohol (122 mg, 0.5 mmol) yielded 117 mg (102%)
   MS (m/e): 350 (M+1)
4-tolualdehyde (41 mg, 0.33 mmol) and 4-amino-1-benzylpiperidine (93 mg, 0.5 mmol) yielded 70 mg (73%)
   MS (m/e): 295 (M+1)
4-tolualdehyde (41 mg, 0,33 mmol) and tetrahydrofurfurylamine (51 mg, 0.5 mmol) yielded 58 mg (86%)
   MS (m/e):206 (M+1)
4-tolualdehyde (41 mg, 0.33 mmol) and α-methyl benzylamine (60 mg, 0.5 mmol) yielded 60 mg (80%)
   MS (m/e): 226 (M+1)

## Claims

1. A scavenger assisted solution phase combinatorial reductive amination process for preparing a library of compounds having a secondary amine scaffold with three variable substituents, wherein each library compound is made in a separate reaction zone and is represented by the formula; 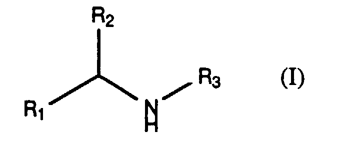 said process comprising the steps of:
a) adding to each reaction zone containing a liquid medium (n) equivalents of a carbonyl functional reactant represented by the formula: 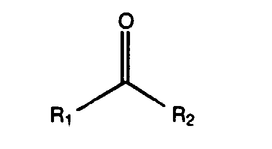 where R₁ is a non-interfering substituent and R₂ is H or a non-interfering substituent;
b) adding to each reaction zone of step (a) at least 1.1(n) equivalents of a solvent soluble primary amine reactant;
R₃-NH₂
where R₃ is a non-interfering substituent and maintaining the reaction zone at a temperature and for a time sufficient to permit reaction;
c) adding to each reaction zone a reducing agent and maintaining each reaction zone at a temperature and for a time sufficient to permit imine reduction;
d) adding to each reaction zone of step (c) a solid supported aldehyde functional scavenger represented by the formula; 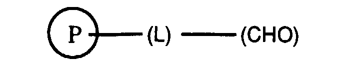 wherein; 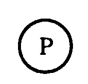 is a solid-support insoluble in the liquid reaction medium of the reaction zone, -(L)- is a divalent linking group, and (CHO) is an aldehyde functional substituent; and adding said scavenger in an amount wherein the equivalents of (CHO) are at least equal to the excess equivalents of unreacted amine reactant used in step (b), and maintaining said reaction zone at a temperature and for a time sufficient to permit reaction of said excess amine reactant and said scavenger;
e) separating the solid supported scavenger from each reaction zone of step (d) and recovering each substantially purified secondary amine library compound.

2. The process of claim 1 wherein the amine reactant of step (b) is selected from the group of aliphatic primary amines having a molecular weight of from 50 to 600.

3. The process of claim 1 wherein from 1.25(n) to 5(n) equivalents of primary amine reactant is used per equivalent of carbonyl functional reactant.

4. The process of claim 1 where the reducing agent of step (c) is a solid supported reducing agent insoluble in the reaction medium.

5. The process of claim 4 wherein the reducing agent is a borohydride functional polymer.

6. The process of claim 1 wherein the solid supported scavenger is separated in step (e) by filtration.

7. The library of secondary amine compounds prepared by the process of claim 1.

8. The individual secondary amine library compounds in the secondary amine library of claim 7.

9. An assay kit for identification of pharmaceutical lead compounds, comprising biological assay materials and wellplate apparatus;
wherein the improvement comprises using as wellplate apparatus a wellplate containing in each well library compound of a diverse combinatorial secondary amine library prepared by the process of claim 1.

10. Wellplate apparatus suitable as a replaceable element in an automated assay machine wherein the improvement comprises,
using as the wellplate apparatus a diverse secondary amine combinatorial wellplate, wherein each well independently contains secondary amine library compound prepared by the processes of claim 1.
